# EUROPEAN PATENT APPLICATION

(11) **EP 4 202 034 A1**
(43) Date of publication of application: **28.06.2023**
(21) Application number: 21882482.9
(22) Date of filing: 14.09.2021
(51) Int. Cl.: C12M 1/34, G01N 1/10, G01N 1/30, G01N 1/31, G01N 1/40, G01N 33/48

(54) **SAMPLE PREPARATION SYSTEM AND SAMPLE PREPARATION METHOD**

(30) Priority: 19.10.2020 JP 2020175426
(71) Applicant: Sony Group Corporation, Minato-Ku, Tokyo, 108-0075 (JP)
(72) Inventor: KATO, Yoshiaki, Tokyo 108-0075 (JP); HOSOKAWA, Hiroyuki, Tokyo 108-0075 (JP); CHUBACHI, Hideya, Tokyo 108-0075 (JP); MACHIDA, Kenzo, Tokyo 108-0075 (JP); FUCHIGAMI, Aya, Tokyo 108-0075 (JP)
(74) Representative: D Young & Co LLP
(86) International application number: PCT/JP2021/033767
(87) International publication number: WO 2022/085341

(57) **Abstract**

An object of the present technology is to provide a sample preparation system for increasing the content percentage of target cells.

The present technology provides a sample preparation system including a bioparticle-capturing module including a substrate to which a substance that captures bioparticles is fixed, a reservoir into which the bioparticles released from the substrate are recovered, and a hollow fiber membrane module through which the bioparticles in the reservoir are flowed. The present technology also provides a sample preparation method including a capturing step of capturing bioparticles with use of a substrate to which a substance that captures the bioparticles is fixed, a recovery step of recovering, into a reservoir, the bioparticles released from the substrate, and a hollow fiber membrane treatment step of causing the bioparticles recovered into the reservoir to flow through a hollow fiber membrane module.

## Description

### [Technical Field]

The present technology relates to a sample preparation system and a sample preparation method, and in particular, to a sample preparation system and a sample preparation method used to prepare a sample that contains bioparticles.

### [Background Art]

Various schemes are used to fractionate or analyze bioparticles. For example, techniques in which bioparticles are fractionated or analyzed in a closed space or an open space have been developed. Typical techniques for fractionation or analysis can include, for example, flow cytometry (hereinafter also referred to as FCM). For fractionation or analysis of bioparticles, cell staining is performed with use of fluorescently labelled antibodies. The cell staining allows a particular cell group to be analyzed and/or fractioned. For example, the use of FITC labelled CD3 antibodies enables T cells to be stained, and the stained T cells can be analyzed and/or fractionated, for example, by FCM. In FCM, all cells are analyzed on a one-by-one basis, enabling very detailed analysis and/or purification.

In bioparticle analysis, such as FCM, in which all cells are analyzed on a one-by-one basis, analysis time correlates with the number of cells to be analyzed. Thus, a sample containing many cells to be analyzed but a small number of target cells can cause an unwanted increase in time required for the bioparticle analysis. In view of this, for example, a sample used for bioparticle analysis can be roughly purified in order to increase the rate of the target cells. The rough purification reduces the number of cells to be analyzed, enabling the analysis time to be shortened.

There have been proposed some techniques that can be associated with such rough purification. For example, PTL 1 listed below describes a "cell separation method of removing red blood cells from a cell suspension containing the red blood cells and white blood cells and recovering white blood cells." PTL 1 states that the method "characterized by including the steps of (A) introducing a cell suspension through an inlet of a cell separating filter obtained by filling a container with a cell separating material and capturing white blood cells in the cell separating filter, (B) introducing a wash liquid through the inlet of the cell separating filter and washing the cell separating filter, and (C) introducing a recovered liquid through an outlet of the cell separating filter and recovering the white blood cells captured in the cell separating filter and in that the wash liquid and the recovered liquid contain a divalent cation chelate agent (concentration of the divalent cation chelate agent in the wash liquid
(W/V)):(concentration of the divalent cation chelate agent in the recovered liquid (W/V)) ranges from 1:200 to 200:1." In addition, NPL 1 listed below describes the use of multilayer polyester nonwoven fibers as a filter to remove red blood cells from umbilical blood.

### [Citation List]

### [Patent Literature]

[PTL 1]
PCT Patent Publication No. WO 2014/119529

[NPL 1]
Sato N, Fricke C, McGuckin C, Forraz N, Degoul O, Atzeni G, Sakurai H., Cord blood processing by a novel filtration system., Cell Prolif., 2015 Dec;48(6):671-81.

### [Summary]

### [Technical Problem]

A sample used for fractionation or analysis of bioparticles desirably has a high content percentage of target particles to be fractionated or analyzed. Thus, the sample can be subjected to treatment for increasing the content percentage of target particles, before the fractionation or analysis. For the treatment, the target particles are required to be selectively and efficiently recovered. In addition, for the treatment, as-needed adjustment of the content percentage of the target particles, for example, concentration of the target particles, is required. Thus, an object of the present technology is to provide a sample preparation system for increasing the content percentage of the target particles.

### [Solution to Problem]

The inventors have found that a particular sample preparation system can solve the above-described problem.

Specifically, the present technology provides a sample preparation system including a bioparticle-capturing module including a substrate to which a substance that captures bioparticles is fixed, a reservoir into which the bioparticles released from the substrate are recovered, and a hollow fiber membrane module through which the bioparticles in the reservoir are flowed.

The sample preparation system may be configured to allow a nucleolytic substance to be fed to the bioparticle-capturing module.

The substance that captures the bioparticles may be fixed to the substrate via nucleic acids.

The sample preparation system may be configured to allow the nucleolytic substance to reach the reservoir or the hollow fiber membrane module through the bioparticle-capturing module.

The sample preparation system may be configured to allow a bioparticle-containing liquid to be fed to the bioparticle-capturing module and to allow a particle-binding substance bound to the bioparticles to be fed to the reservoir.

The particle-binding substance may be an antibody.

The bioparticles may be cells.

The sample preparation system may include a circulating channel through which the bioparticles are circulated between the reservoir and the hollow fiber membrane module.

The sample preparation system may further include a branching channel branching off from the circulating channel and leading to the reservoir.

The sample preparation system may further include an analysis apparatus that analyzes contents of the reservoir.

The sample preparation system can control a flow-through operation in which the bioparticles are flowed through the hollow fiber membrane module, on the basis of an analysis result from the analysis apparatus.

The sample preparation system may include a pump provided on a channel leading from the bioparticle-capturing module to the reservoir.

The pump may be a tube pump.

The sample preparation system may be configured to allow the bioparticles released from the substrate to be fed to the reservoir by driving of the pump.

The sample preparation system may be configured to allow a particle-binding substance bound to the bioparticles to be fed to the reservoir by driving of the pump.

A bioparticle circulation pump may be provided on a channel leading from the reservoir to the hollow fiber membrane module.

The hollow fiber membrane module may include a discharge channel through which a liquid separated from the bioparticles is discharged, and a discharge pump may be provided on the discharge channel.

The sample preparation system may be configured to prevent a sample containing the bioparticles from communicating fluidly with an external environment.

In addition, the present technology provides a sample preparation method including a capturing step of capturing bioparticles with use of a substrate to which a substance that captures the bioparticles is fixed, a recovery step of recovering, into a reservoir, the bioparticles released from the substrate, and a hollow fiber membrane treatment step of causing the bioparticles recovered into the reservoir to flow through a hollow fiber membrane module.

### [Brief Description of Drawings]

[FIG. 1]

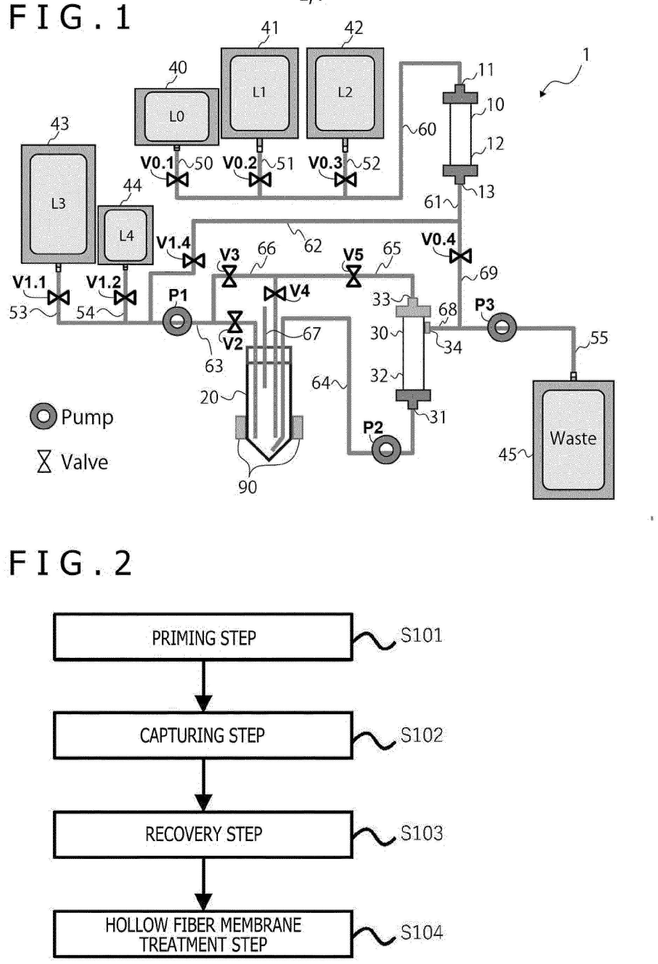

   FIG. 1 is a schematic diagram depicting a configuration example of a sample preparation system of the present technology.
[FIG. 2]
   FIG. 2 is an example of a flowchart of a sample preparation method using the sample preparation system of the present technology.
[FIG. 3]

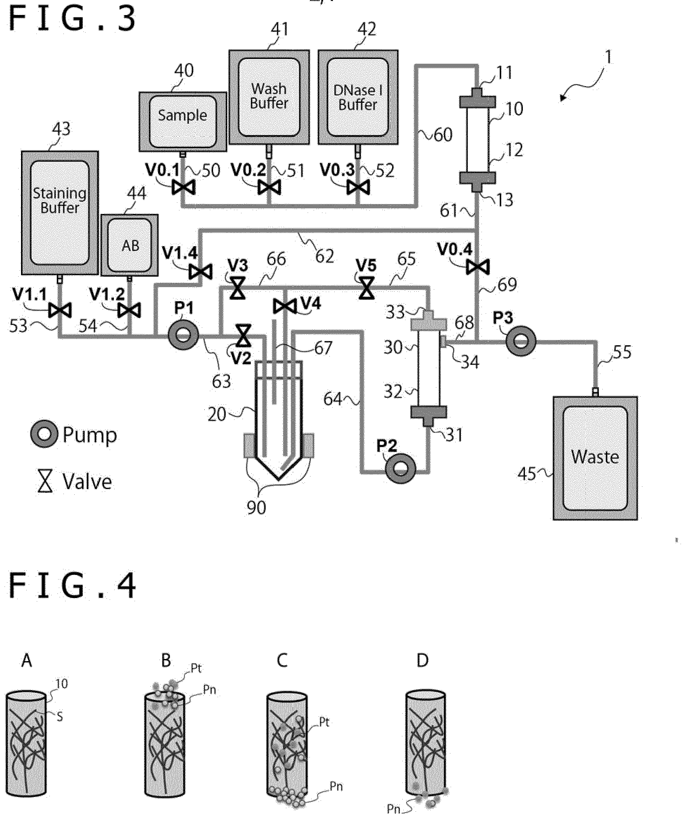

   FIG. 3 is a schematic diagram depicting a configuration example of the sample preparation system of the present technology.
[FIG. 4]
   FIG. 4 is a schematic diagram illustrating capturing and releasing of target cells by a bioparticle-capturing module.
[FIG. 5]

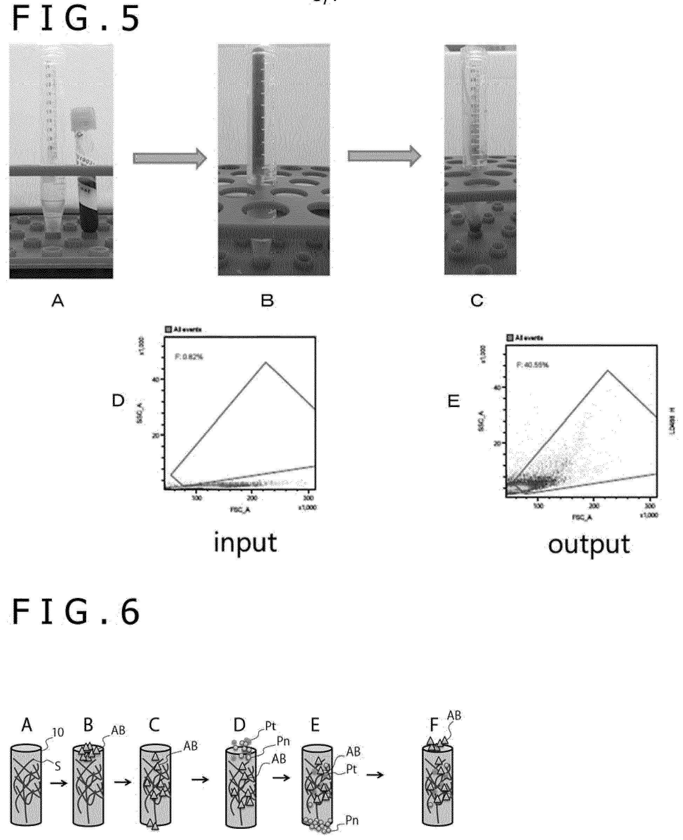

   FIG. 5 is a diagram depicting results of treatment of blood using a substrate to which a bioparticle-capturing substance is fixed.
[FIG. 6]
   FIG. 6 is a schematic diagram illustrating capturing and releasing of the target cells by the bioparticle-capturing module.
[FIG. 7]

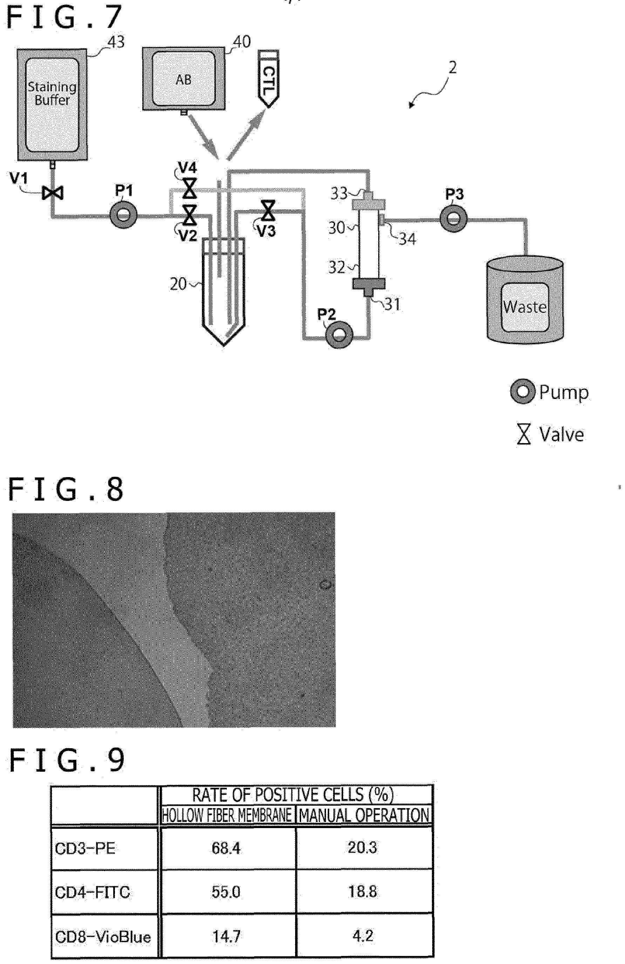

   FIG. 7 is a schematic diagram of an apparatus prepared to confirm a clogging prevention effect in a hollow fiber membrane treatment step.
[FIG. 8]
   FIG. 8 is a diagram depicting a photograph of cellular aggregates formed in a reservoir.
[FIG. 9]
   FIG. 9 is a diagram depicting results of analysis by a cell analyzer.
[FIG. 10]

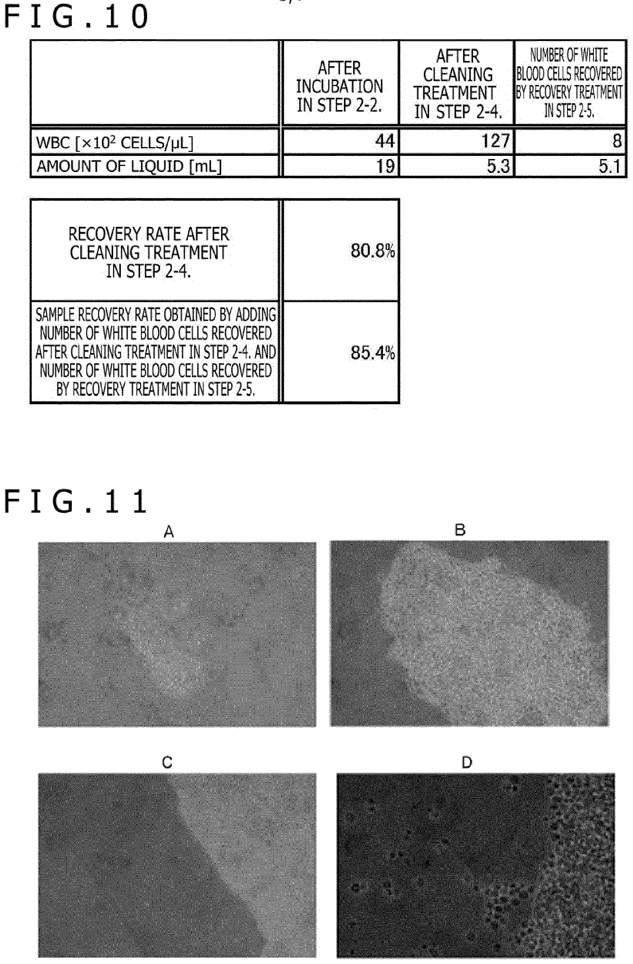

   FIG. 10 is a diagram depicting measurement results for the number of cells counted by a blood cell counting apparatus.
[FIG. 11]
   FIG. 11 is a diagram depicting photographs of cell aggregates.
[FIG. 12]

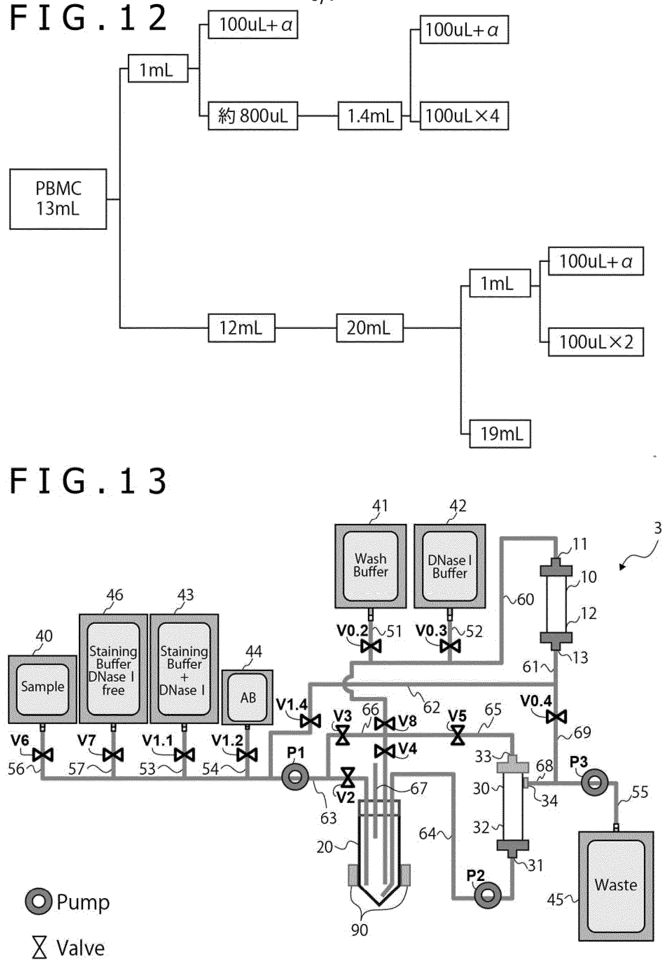

   FIG. 12 is a schematic diagram of a staining procedure.
[FIG. 13]
   FIG. 13 is a schematic diagram depicting a configuration example of the sample preparation system of the present technology.
[FIG. 14]

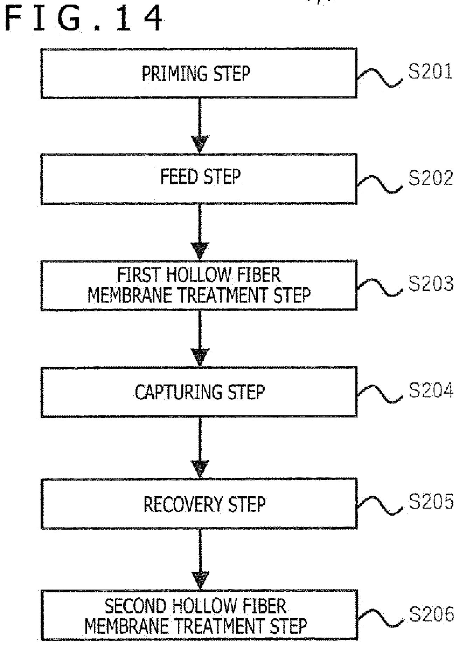

   FIG. 14 is an example of a flowchart of the sample preparation method using the sample preparation system of the present technology.

### [Description of Embodiments]

Preferred modes for implementing the present technology will be described. Note that embodiments described below illustrate typical embodiments of the present technology and that the scope of the present technology is not limited only to these embodiments. Note that the present technology will be described in the following order.
1. First Embodiment (Sample Preparation System)
   (1) Description of First Embodiment
   (2) Configuration Example of Sample Preparation System according to Present Technology
   (3) Example of Sample Preparation Method Using Sample Preparation System according to Present Technology
      (3-1) Priming Step
      (3-2) Capturing Step
      (3-3) Recovery Step
      (3-4) Hollow Fiber Membrane Treatment Step
      (3-5) Modification
   (4) Example of Treatment Using Bioparticle-Capturing Module
      (4-1) Configuration Example and Operation Example of Bioparticle-Capturing Module
      (4-2) Test Example (Treatment of Blood with Use of Substrate to Which Bioparticle-Capturing Substance Is Fixed)
      (4-3) Another Configuration Example and Another Operation Example of Bioparticle-Capturing Module
   (5) Evaluation of Effects of Nucleolytic Substance on Treatment Using Hollow Fiber Membrane Module
   (6) Evaluation of Antibody Staining Using Hollow Fiber Membrane Module
2. Second Embodiment (Sample Preparation Method)

### 1. First Embodiment (Sample Preparation System)

### (1) Description of First Embodiment

A sample preparation system of the present technology includes a bioparticle-capturing module including a substrate to which a substance that captures bioparticles is fixed, a reservoir into which the bioparticles released from the above-described substrate are recovered, and a hollow fiber membrane module through which the bioparticles in the reservoir are flowed. The above-described bioparticle-capturing module can selectively capture target bioparticles. Then, the bioparticles released from the above-described substrate of the above-described bioparticle-capturing module are recovered to the above-described reservoir. The bioparticles recovered into the reservoir are flowed through the above-described hollow fiber membrane module. Thus, the target bioparticles can be selectively and efficiently recovered, and further, the concentration of the bioparticles can be adjusted.

### (2) Configuration Example of Sample Preparation System according to Present Technology

A configuration example of the sample preparation system of the present technology will be described below with reference to FIG. 1.

A sample preparation system 1 depicted in FIG. 1 includes a bioparticle-capturing module 10, a reservoir 20, and a hollow fiber membrane module 30.

The bioparticle-capturing module 10 includes an inlet 11, a container 12, and an outlet 13. The container 12 is packed with a substrate to which a substance that captures bioparticles is fixed.

The bioparticle-capturing module 10 may be configured to allow a bioparticle-containing liquid to be fed from the inlet 11 into the container 12. The bioparticle-containing liquid may contain target particles and non-target particles.

For example, as depicted in FIG. 1, the bioparticle-capturing module 10 is connected through channels 50 and 60 to a container 40 (for example, a bag) filled with a bioparticle-containing liquid L0. More specifically, an outlet of the container 40 and the inlet 11 are connected to each other through the channels 50 and 60 to allow the bioparticle-containing liquid L0 in the container 40 to be fed into the container 12.

In addition, the bioparticle-capturing module 10 is also connected through a channel 51 and the channel 60 to a container 41 (for example, a bag) filled with a wash liquid L1 (particularly, a wash buffer). More specifically, an outlet of the container 41 and the inlet 11 are connected to each other through the channels to allow the wash liquid L1 in the container 41 to be fed into the container 12.

In addition, the bioparticle-capturing module 10 is also connected through a channel 52 and the channel 60 to a container 42 (for example, a bag) filled with a nucleolytic substance-containing liquid L2 (particularly, a nucleolytic substance-containing buffer). More specifically, an outlet of the container 42 and the inlet 11 are connected to each other through the channels to allow the nucleolytic substance-containing liquid L2 in the container 42 to be fed into the container 12.

A nucleolytic substance contained in the above-described nucleolytic substance-containing liquid may be, for example, a nucleolytic enzyme, and may particularly be deoxyribonuclease, ribonuclease, or both of these. The above-described nucleolytic enzyme may be of, for example, an endo type, an exo type, or both of these. Preferably, the above-described nucleolytic substance may contain deoxyribonuclease, and may particularly contain deoxyribonuclease and need not contain ribonuclease. An example of deoxyribonuclease can include, for example, DNaseI.

The inventors have found that a liquid containing the nucleolytic substance, particularly, the nucleolytic enzyme, and more particularly, deoxyribonuclease is suitable for preventing clogging in the sample preparation system of the present technology, and particularly, for preventing clogging. Clogging can occur, for example, in various channels or in the hollow fiber membrane module described below. In particular, clogging is likely to occur in treatment using the hollow fiber membrane module described below, and particularly, in a case where bioparticles, for example, cells or the like are washed or concentrated by the hollow fiber membrane module. In the present technology, the use of the above-described nucleolytic substance allows possible clogging to be prevented.

The clogging is considered to be due to, for example, DNAs released from dead cells. In the present technology, it is considered that the DNAs are decomposed by causing the liquid containing the above-described nucleolytic substance to pass through the various channels, the bioparticle-capturing module, the reservoir, and the hollow fiber membrane module, allowing clogging to be prevented.

As described above, the sample preparation system of the present technology may be configured to allow the nucleolytic substance to be fed to the above-described bioparticle-capturing module. More preferably, the above-described sample preparation system may be configured to allow the above-described nucleolytic substance to reach the above-described reservoir and/or the above-described hollow fiber membrane module through the above-described bioparticle-capturing module.

As depicted in FIG. 1, the channels 50, 51, and 52 are connected to the channel 60.

Valves V0.1, V0.2, and V0.3 are respectively provided on the channels 50, 51, and 52.

Opening or closing the valve V0.1 enables or disables feeding, to the container 12, of the bioparticle-containing liquid L0 contained in the container 40.

Opening or closing the valve V0.2 enables or disables feeding, to the container 12, of the wash liquid L1 contained in the container 41.

Opening or closing the valve V0.3 enables or disables feeding, to the container 12, of the nucleolytic substance-containing liquid L2 contained in the container 42.

The channels 50, 51, 52, and 60 may be, for example, tubes. Materials for the tubes may be selected as appropriate by a person skilled in the art. In addition, the dimensions of cross sections of the channels may be set as appropriate by the person skilled in the art, depending on the bioparticle-containing liquid to be flowed through the channels. Other channels depicted in FIG. 1 may also be tubes, and the materials and cross-sectional dimensions of these channels may similarly be selected as appropriate by the person skilled in the art.

The container 12 of the bioparticle-capturing module 10 is packed with a substrate to which a substance that captures bioparticles is fixed. The shape of the container 12 may be selected as appropriate by the person skilled in the art, and can be, for example, columnar, that is, cylindrical. The column shape preferably reduces the effect of hydraulic pressure on the container shape.

In the present specification, the substance that captures the bioparticles may mean a substance used to capture the bioparticles. The substance that captures the bioparticles may be a substance that itself binds to the bioparticles (in the present specification, also referred to as a "bioparticle-binding substance") or a substance that captures the bioparticles via a different substance. In the latter case, the substance that captures the bioparticles is not the substance that itself binds to the bioparticles, and the different substance may be a substance that binds to the bioparticles.

The above-described bioparticle-binding substance may be a substance that itself binds to the bioparticles as described above, may be, for example, antibodies or antibody fragments, may more particularly be antibodies or antibody fragments that bind to antigens present on the surfaces of the bioparticles, and may much more particularly be antibodies or antibody fragments that bind to surface antigens of cells.

The substance that captures the bioparticles via the above-described different substance need not itself be a substance that binds to the bioparticles, as described above. The substance that captures the bioparticles via the above-described different substance may be, for example, a substance that binds to the bioparticle-binding substance, may particularly be a protein that binds to antibodies or antibody fragments, and may more particularly be a protein that specifically binds to antibodies or antibody fragments. An example of the protein can be an antibody-binding protein. The antibody-binding protein may be, for example, any one or two or more of a protein A, a protein G, a protein L, and a protein A/G.

The type of the substance that captures the above-described bioparticles may be selected as appropriate by the person skilled in the art, depending on the type of the bioparticles to be recovered (particularly, the substance present on the surfaces of the bioparticles).

In the present specification, the bioparticles may be biological particles and may mean, for example, particles constituting a living organism. The bioparticles may be microparticles.

The bioparticles may be, for example, cells. The cells can include animal cells (hemocyte-related cells and the like) and plant cells. The cells can particularly be hematological cells or tissue cells. The above-described hematological cells can include, for example, white blood cells (for example, peripheral-blood mononuclear cells), red blood cells, and platelets, and the above-described hematological cells particularly include white blood cells. The white blood cells can be, for example, monocytes (macrophage), lymphocytes, neutrophils, basophils, and eosinophils. The cells may be floating cells, for example, T cells, B cells, and the like. The above-described tissue cells may be, for example, adhesive cultured cells, adhesive cells peeled off from tissues, or the like. In addition, the cells may be tumor cells. The cells may be cultured or may not be cultured. The bioparticles may be cell masses, for example, spheroids and organoids.

The bioparticles may be non-cellular biotic components, for example, extracellular vesicles, particularly, exosomes or microvesicles.

The bioparticles may be microorganisms or viruses. The microorganisms can include bacteria such as Escherichia coli and fungi such as yeast. The viruses may be, for example, DNA viruses or RNA viruses, and may be viruses having an envelope or no envelope.

The bioparticles can encompass biological polymers such as nucleic acids, proteins, and complexes thereof. The biological polymers may be, for example, extracted from cells or contained in a blood sample or any other liquid sample.

In the present specification, the bioparticle-containing liquid may be a liquid obtained from a living organism, for example, a body fluid. The body fluid may be blood, lymph, a tissue fluid (for example, an interstitial fluid, an intercellular fluid, an extracellular fluid, and the like), or a coelomic fluid (for example, a chorionic cavity fluid, pleural effusion, an ascites fluid, pericardial effusion, a cerebrospinal fluid (spinal fluid), a joint fluid (synovial fluid), or the like). In addition, the bioparticle-containing liquid may be a liquid obtained from the above-described body fluids.

In an embodiment of the present technology, the above-described bioparticle-containing liquid may be a blood sample and may particularly be a sample that contains white blood cells. The bioparticle-containing liquid may particularly be a blood sample subjected to red blood cell separation treatment. Note that red blood cells need not completely be removed from the blood sample but that the blood sample may contain red blood cells. For example, the blood sample of blood collected from the living organism may have a reduced amount of red blood cells due to the separation treatment.

The above-described substrate may include a material through which the above-described bioparticle-containing liquid can pass, and may be, for example, a porous material, a fibrous material, or a beads like material. The substrate including a material having such a shape or structure has a large surface area and allows many substances capturing the bioparticles to be fixed to the substrate. The substrate further allows the above-described bioparticle-containing liquid to pass through.

The material of the above-described substrate may be, for example, a proteinaceous material, an organic polymer-based material, or an inorganic material. The above-described proteinaceous material can be collagen or egg white. The above-described organic polymer-based material can include, for example, acrylamide, polystyrene, polycaprolactone, and lactic acid-based polymer. The above-described inorganic material can be, for example, titanium, particularly, titanium fibers.

The above-described substrate may include, for example, a spongelike material, and may include, for example, a collagen sponge.

The above-described substrate may include, for example, a cryogel material, and may include, for example, cryogel of acrylamide or egg white.

The above-described substrate may include, for example, a scaffold material, and may include a scaffold material including, for example, polystyrene, polycaprolactone, a lactic acid-based polymer or a combination of a lactic acid-based polymer and hydroxyapatite, or titanium fibers.

The bioparticle-capturing module 10 may be configured to be capable of discharging a liquid through the outlet 13.

For example, as illustrated in FIG. 1, the bioparticle-capturing module 10 may be configured to cause a liquid discharged through the outlet 13 (the liquid containing the bioparticles captured by the substance that captures the bioparticles and subsequently released from the substance) to be fed to the reservoir 20. For feeding of the liquid to the reservoir 20, the outlet 13 may be connected to the reservoir 20 via a channel. More specifically, a channel 61 is connected to the outlet 13, the channel 61 is connected to a channel 62, the channel 62 is connected to a channel 63, and the channel 63 is connected to the reservoir 20. That is, the outlet 13 is connected to the reservoir 20 via the channels 61, 62, and 63.

In addition, for example, as illustrated in FIG. 1, the bioparticle-capturing module 10 may be configured to cause waste discharged through the outlet 13 to be recovered into a waste recovery container 45. To enable waste to be recovered, the channel 61 branches into two channels, i.e., the channel 62 and a channel 69, and of the two channels, the channel 69 is used to recover waste. The channel 69 is connected to a channel 55, and the channel 55 is connected to the waste recovery container 45. That is, the outlet 13 is connected to the waste recovery container 45 via the channels 61, 69, and 55.

A valve V0.4 is provided on the channel 69. Opening or closing the valve V0.4 enables or disables recovery of waste into the waste recovery container 45 with use of the channel 69.

The reservoir 20 is connected via the channels 54 and 63 to a container 44 (particularly, a bag) containing a particle binding substance-containing liquid L4. More specifically, an outlet of the container 44 and the reservoir 20 are connected to each other through the tubes 54 and 63 to allow the particle binding substance-containing liquid in the container 44 to be fed into the reservoir 20. The particle binding substance-containing liquid L4 may be a liquid used to bind the particle-binding substance to the bioparticles. The particle-binding substance may be, for example, an antibody, particularly, a fluorescently labelled antibody or the like. The type of the particle-binding substance and the composition of the liquid can be selected as appropriate by the person skilled in the art, depending on the sample to be prepared.

The reservoir 20 is connected through a channel 53 and the channel 63 to a container 43 (particularly, a bag) containing a binding auxiliary liquid L3. More specifically, the outlet of the container 43 and the reservoir 20 are connected to each other through the channels 53 and 63 to allow the binding auxiliary liquid L3 in the container 43 to be fed into the reservoir 20. The above-described binding auxiliary liquid may mean a liquid used to bind the particle-binding substance (for example, the antibodies, particularly, the fluorescently labelled antibodies) to the bioparticles.

For example, the above-described binding auxiliary liquid may be a buffer used to stain the bioparticles with the antibodies and may be what is generally called a staining buffer. The buffer may be used to, for example, dilute the antibodies or remove extra antibodies after labelling of the antibodies. The staining buffer may be, for example, a PBS containing a protein (for example, a serum protein) or a normal saline solution. The protein is added to prevent nonspecific binding of the antibodies, and, for example, FBS or albumin (BSA or the like) can be added to the normal saline solution.

As illustrated in FIG. 1, the channels 53, 54, and 62 are connected to the channel 63.

In addition, valves V1.1, V1.2, and V1.4 are respectively provided on the channels 53, 54, and 62.

Opening or closing the valve V1.1 enables or disables feeding of the binding auxiliary liquid L3 contained in the container 43 to be fed to the reservoir 20.

Opening or closing the valve V1.2 enables or disables feeding of the particle binding substance-containing liquid L4 contained in the container 44 to be fed to the reservoir 20.

Opening or closing the valve V1.4 enables or disables feeding of the liquid contained in the container 12 to be fed to the reservoir 20.

A pump P1 is provided on the channel 63. The pump P1 may be, for example, a tube pump and more preferably, may be a peristaltic pump. Such a pump is suitable for liquid delivery for capturing of the bioparticles by the bioparticle-capturing module and for liquid delivery for discharging of the bioparticles released from the substrate. Driving of the pump P1 and control of opening and closing of the various valves enable feeding of the various liquids to the bioparticle-capturing module 10 and discharging of the liquid from the bioparticle-capturing module 10 as well as feeding of the various liquids to the reservoir 20. These liquid delivery operations will be described below.

As described above, the present technology may provide the pump on the channel leading from the above-described bioparticle-capturing module to the above-described reservoir. The sample preparation system of the present technology may be configured to allow the bioparticles released from the above-described substrate to be fed to the above-described reservoir by driving of the pump. Further, the sample preparation system of the present technology may be configured to allow the particle-binding substance bound to the bioparticles to be fed to the above-described reservoir by driving of the above-described pump.

The hollow fiber membrane module 30 includes an inlet 31, a container 32, an outlet 33, and a waste outlet 34. A hollow fiber membrane is packed into the container 32.

The hollow fiber membrane included in the hollow fiber membrane module 30 may be selected by the person skilled in the art, depending on the size and type of the bioparticles to be concentrated. The hollow fiber membrane may include, for example, mPES (modified polyether sulfone, ME (mixed cellulose ester), PES (polyether sulfone), or PS (polysulfone). A pore size of the hollow fiber membrane represented as MWCO (Molecular Weight Cut Off) is, for example, 1 kD or more and 1000 kD or less, particularly, 2 kD or more and 900 kD or less, and more particularly, 3 kD or more and 800 kD or less. The pore size of the hollow fiber membrane module is, for example, 0.1 µm or more and 1.0 µm or less, particularly, 0.15 µm or more and 0.9 µm or less, more particularly, 0.2 µm or more and 0.8 µm or less.

The hollow fiber membrane module 30 is configured to allow the bioparticle-containing liquid in the reservoir 20 to pass through the hollow fiber membrane module 30. For example, the hollow fiber membrane module 30 may be configured to allow the bioparticle concentration of the bioparticle-containing liquid in the reservoir 20 to be adjusted, and may be configured to, for example, allow the bioparticle concentration to be increased or reduced.

For example, as illustrated in FIG. 1, the hollow fiber membrane module 30 is connected to the reservoir 20 through the channels 64 and 65. The channels 64 and 65 are collectively referred to as a circulating channel.

The liquid in the reservoir 20 (particularly, the bioparticle-containing liquid) may be fed to the hollow fiber membrane module 30 through the channel 64, and the bioparticle-containing liquid having passed through the hollow fiber membrane module 30 may be fed to the reservoir 20 through the channel 65. The bioparticles thereby circulate between the reservoir 20 and the hollow fiber membrane module 30.

As described above, the sample preparation system of the present technology may preferably include a circulating channel through which the bioparticles circulate between the reservoir 20 and the hollow fiber membrane module 30.

Note that the circulation may be in the opposite direction. That is, the liquid in the reservoir 20 (particularly, the bioparticle-containing liquid) may be fed to the hollow fiber membrane module 30 through the channel 65, and the bioparticle-containing liquid having passed through the hollow fiber membrane module 30 may be fed to the reservoir 20 through the channel 64.

A pump P2 is provided on the channel 64. The pump P2 may be, for example, a tube pump and may more preferably be a peristaltic pump. Such a pump is suitable for liquid delivery for treatment performed by the hollow fiber membrane module. As described above, in the present technology, the bioparticle circulating pump may be provided on the channel leading from the reservoir 20 to the hollow fiber membrane module 30.

Control of driving of the pumps P1, P2, and P3 and control of opening and closing of the various valves enable feeding of the various liquids to the reservoir 20 and the hollow fiber membrane treatment. The details of these liquid delivery operations will be described below.

Two valves V4 and V5 are provided on the channel 65. Opening or closing the valves V4 and V5 enables or disables flow-through of the liquid between the reservoir 20 and the hollow fiber membrane module 30 (particularly, the container 32).

Note that a single valve may be provided but that two valves are preferably provided in order to perform operations using a branching channel 66 described below.

On the channel 65, the branching channel 66 that branches from between the valves V4 and V5 on the channel 65 is provided. The branching channel 66 extends from the channel 65 to the channel 63 and is connected to the channel 63 at a position between the pump P1 on the channel 63 and the valve V2. That is, the branching channel 66 includes one end at the position between the pump P1 on the channel 63 and the valve V2 and the other end between the valve V4 and the valve V5 on the channel 65.

The branching channel 66 branches from the circulating channel and can also be said to lead to the reservoir 20 via the channel 63. As described above, the sample preparation system of the present technology preferably includes the branching channel branching off from the above-described circulating channel and leading to the above-described reservoir.

The branching channel 66 is used for dead volume recovery treatment as described below.

A valve V3 is provided on the branching channel 66. Opening or closing the valve V3 enables or disables flow of the liquid from the channel 65 to the channel 63 or the opposite flow from the channel 63 to the channel 65.

A channel 68 through which waste (which is also represented as permeate in the hollow fiber membrane treatment) flows is connected to the hollow fiber membrane module 30, the waste resulting from concentration of the bioparticles by the module. The channel 68 may lead to the waste recovery container. For example, as illustrated in FIG. 1, the outlet 34 through which the waste is discharged is connected to the channel 68, and the channel 68 is further connected to the waste recovery container 45 via the channel 55. Thus, the channel 68 joins with a channel 67 through which the waste from the bioparticle-capturing module flows, and is connected to the channel 55. As described above, the sample preparation system of the present technology may be configured to cause the waste from the above-described bioparticle-capturing module and the waste from the above-described hollow fiber membrane module to be recovered into one waste recovery container.

A pump P3 is provided on the channel 55. The pump P3 may be, for example, a tube pump and may more preferably be a peristaltic pump. Such a pump is suitable for waste recovery from the bioparticle-capturing module or the hollow fiber membrane module. By driving the pump P3 in combination with the pump P1 and/or the pump P2 and controlling opening and closing of the various valves, for example, waste recovery is enabled. The details of these liquid delivery operations will be described below

As described above, in the present technology, the hollow fiber membrane module may include the discharging channel (corresponding to the channels 68 and 55) through which the liquid separated from the bioparticles is discharged, and the discharging pump may be provided on the discharging channel.

The liquid recovery channel 67 may be connected to the reservoir 20. The liquid recovery channel 67 may be used to collect, from the reservoir 20, the bioparticle-containing liquid that has not yet been subjected to, is being subjected to, or has been subjected to the treatment using the hollow fiber membrane module (particularly, bioparticle concentration treatment).

In addition, the liquid recovery channel 67 may be used to collect, from the reservoir 20, for example, the bioparticle-containing liquid that has not yet been subjected to, the bioparticle-containing liquid that is being subjected to, or the bioparticle-containing liquid that has been subjected to treatment for binding, to the bioparticles, the particle-binding substance contained in the container 44.

The reservoir 20 may include an analysis apparatus 90. The analysis apparatus 90 analyzes contents (particularly, the bioparticle-containing liquid) of the reservoir 20. For example, the analysis apparatus 90 may be configured as a concentration measurement apparatus that measures the concentration of the bioparticle-containing liquid. The concentration measurement apparatus can measure the concentration of the bioparticle-containing liquid by measuring, for example, the turbidity of the bioparticle-containing liquid or measuring fluorescence resulting from the bioparticle-containing liquid. Depending on an analysis result produced by the analysis apparatus 90, for example, the bioparticle concentration treatment using the hollow fiber membrane module 30 is continued or stopped to allow the bioparticle-containing liquid in the reservoir 20 to be adjusted to a desired concentration.

The sample preparation system of the present technology can further include a control section (not depicted in the figures) that controls the operation of each of the elements constituting the system. The control section can, for example, control the operation of the group of pumps and/or the group of valves described above. For example, the control section may control the operation of the group of pumps and/or the group of valves described above, according to a predetermined program.

In addition, the control section may be configured to receive analysis results from the analysis apparatus 90. The control section may control the operation of the group of pumps and/or the group of valves described above, according to an analysis result from the analysis apparatus 90. For example, in response to reception of a predetermined analysis result, the control section may control driving of any one or two or more pumps of the above-described group of pumps, and can particularly start or stop the driving. In addition, in response to reception of a predetermined analysis result, the control section may control opening and closing of any one or two or more valves of the above-described group of valves. Thus, on the basis of the analysis result from the above-described analysis apparatus, the sample preparation system of the present technology can control various steps included in a sample preparation method described below and can control, for example, the start or end of the various steps.

For example, the sample preparation system (particularly, the control section) of the present technology is configured to allow a priming step S101, a capturing step S102, a recovery step S103, and a hollow fiber membrane treatment step S104 described below to be controlled, and may be configured to, for example, allow any one or more of these steps to be controlled.

Preferably, the sample preparation system of the present technology may be configured to control, on the basis of the analysis result from the analysis apparatus, a flow-through operation in which the bioparticles flow through the hollow fiber membrane module. More specifically, the sample preparation system (particularly, the control section) of the present technology may control the hollow fiber membrane treatment step S104 described below and various treatment operations included in this step and can particularly control this step or the start or end of various treatment operations included in this step. Thus, for example, once the bioparticle-containing liquid in the reservoir reaches a desired concentration, the concentration adjustment treatment or the concentration treatment can be automatically ended.

The control section may be configured as an information processing apparatus (computer), and, for example, a general-purpose computer can implement functions of the control section.

Preferably, the sample preparation system of the present technology is configured to prevent a sample containing the bioparticles from communicating fluidly with an external environment. For example, as seen from FIG. 1, a sample preparation system 1 depicted in FIG. 1 is configured to prevent the external environment from being brought into contact with a space through which the bioparticle-containing liquid contained in the container 40 passes before the bioparticle-containing liquid is recovered into the reservoir 20 through the bioparticle-capturing module 10 and a space through which the liquid between the reservoir 20 and the hollow fiber membrane module 30 passes. As described above, the sample preparation system of the present technology can execute a series of processes of preparing a target sample from the bioparticle-containing liquid, without contact with the external environment (for example, outside air or liquid), that is, can execute the processes in a closed manner. Thus, the sample can be prevented from being contaminated.

The sample prepared by the sample preparation system of the present technology may be used as a sample used for fractionation or analysis of the bioparticles. For example, the sample prepared by the sample preparation system of the present technology may be used as a sample provided to an apparatus that fractionates or analyze the bioparticles in a closed space or may be used as a sample provided to an apparatus that fractionates or analyzes the bioparticles in an open space. The apparatus that fractionates or analyzes the bioparticles in the above-described closed space can be, for example, a microparticle fractionation apparatus described in JP 2020-76736A, but the present technology is not limited to this apparatus. The apparatus that fractionates or analyzes the bioparticles in the above-described open space can be, for example, a microparticle measurement apparatus described in JP 2020-51936A, but the present technology is not limited to this apparatus. The sample preparation system of the present technology may be used to prepare a sample provided to such an apparatus for fractionation or analysis.

### (3) Example of Sample Preparation Method Using Sample Preparation System according to Present Technology

An example of the sample preparation method using the sample preparation system according to the present technology will be described below with reference to FIGS. 2 and 3. In this example, executed are treatment for selectively recovering, into the reservoir, target cells from a cell-containing liquid containing the target cells and nontarget cells, by the bioparticle-capturing module and treatment for increasing the concentration of the target cells in the reservoir by the hollow fiber membrane module. In this example, treatment in which the target cells are stained with fluorescently labelled antibodies is further executed.

The cell-containing liquid may be, for example, a blood sample, and may particularly be a sample that contains white blood cells. The cell-containing liquid may particularly be a blood sample that has been subjected to red blood cell separation treatment. Note that the red blood cells need not completely be removed from the blood sample but that the blood sample may contain red blood cells. For example, the blood sample of blood collected from the living organism may have a reduced amount of red blood cells due to the separation treatment.

FIG. 2 is an example of a flow of the sample preparation method. As depicted in FIG. 2, the sample preparation method includes, for example, the priming step S101, the capturing step S102, the recovery step S103, and the hollow fiber membrane treatment step S104. These steps will be described below with also reference to FIG. 1.

FIG. 3 corresponds to a figure of the configuration example illustrated in FIG. 1, the figure depicting, in the containers, the respective types of the various liquids used in the present example. As depicted in FIG. 3, the container 41 contains a wash buffer. The container 42 contains a DNaseI-containing buffer as a nucleolytic substance-containing liquid. The container 43 contains a staining buffer as a binding auxiliary liquid. The container 44 contains a fluorescently labelled antibodycontaining liquid as a particle binding substance-containing liquid.

The container 12 of the bioparticle-capturing module 10 is packed with a substrate on which antibodies that specifically bind to target cells are in a solid phase. The antibodies correspond to the substance that captures the bioparticles in the present technology. The antibodies are fixed to the substrate via DNAs, that is, the DNAs are bound to the substrate, and the antibodies are bound to the DNAs. Thus, cutting off or degrading the DNAs releases the antibodies from the substrate.

### (3-1) Priming Step

In the priming step S101, the channel through which the cell-containing liquid passes is filled with, for example, the wash buffer or the staining buffer. In the priming step S101, the bioparticle-capturing module 10, the reservoir 20, and the hollow fiber membrane module 30 can also be filled with the wash buffer or the staining buffer. This suppresses clogging of the channel due to nonspecific binding between the channel and cells.

For example, controlling the valves and the pumps as described below can cause priming using the wash buffer.

Note that the control of the valves and the pumps described below may be performed by a user or may be performed by the sample preparation system of the present technology itself (particularly, the above-described control section). In addition, the user or the sample preparation system of the present technology itself (particularly, the above-described control section) may also perform the control of the valves and the pumps in the priming step and the following steps (and the various treatment operations included in the steps).

The sample preparation system of the present technology itself, particularly, the above-described control section, controls the valves and the pumps to enable automatic sample preparation.

For example, with the valves V0.2, V1.4, and V2 open, the pump P1 is driven. Thus, the channel 60, the bioparticle-capturing module 10, the channels 61, 62, and 63, and the reservoir 20 are primed with use of the wash buffer.

Next, with the valves V0.2 and V0.4 open, the pump P3 is driven. Thus, the channels 69 and 55 are primed with use of the wash buffer.

Then, with the valves V4 and V5 open and with the valves V2 and V3 closed, the pumps P2 and P3 are driven. Thus, the channels 64, 65, 68, and 55 and the hollow fiber membrane module 30 are primed with use of the wash buffer.

Subsequently, with the valves V2, V3, and V5 open and with the valve V4 closed, the pump P2 is driven. Thus, the channel 66 is primed with use of the wash buffer.

Note that, by changing the manner of controlling the pumps and the valves, for example, the channels 64, 65, and 66 and the like can also be primed with use of the staining buffer.

### (3-2) Capturing Step

### (3-2-1) Capturing Treatment

In the capturing step S102, the cell-containing liquid in the container 40 is fed to the bioparticle-capturing module 10. In the container 12 of the bioparticle-capturing module 10, the antibodies that specifically bind to the target cells are in the solid phase. Thus, the target cells bind to the antibodies and are captured into the container 12.

For feeding of the above-described cell-containing liquid to the bioparticle-capturing module 10, for example, the pump P3 is driven with the valves V0.1 and V0.4 open. During the driving, the other valves may be closed, or the other pumps need also not be driven.

The above-described driving causes the above-described cell-containing liquid to flow from the inlet 11 of the bioparticle-capturing module 10 into the container 12 through the channels 50 and 60. Thus, the target cells are captured by the antibodies in the container 12 as described above, while the nontarget cells pass through the container 12 and flow out through the outlet 13. The target cells then pass through the channels 61, 69, and 55 and are recovered into the waste container 45.

### (3-2-2) Surplus Cell Removal Treatment

In the operation in (3-2-1) described above, some of the nontarget cells are recovered into the waste container 55, while some nontarget cells that are not bound to the above-described antibodies but remain in the container 12 of the bioparticle-capturing module 10 can be present. Thus, to remove the nontarget cells from the container 12, surplus cell removal treatment using the wash buffer may be executed. The removal treatment may be executed by causing the wash buffer in the container 41 to pass through the bioparticle-capturing module 10.

For the above-described removal treatment, for example, the pump P3 is driven with the valves V0.2 and V0.4 open. During the driving, the other valves may be closed, or the other pumps need also not be driven.

The above-described driving causes the above-described wash buffer to flow from the inlet 11 of the bioparticle-capturing module 10 into the container 12 through the channels 51 and 60, then passes through the container 12, and flows out through the outlet 13. Thus, the nontarget cells flow out through the outlet 13 of the container 12 and are then recovered into the waste container 55 through the channels 61, 69, and 55.

### (3-3) Recovery Step

In the recovery step S103, the target cells in the container 12 of the bioparticle-capturing module 10 are released from the substance that captures the bioparticles and recovered into the reservoir 20. To perform the above-described release, the DNaseI-containing buffer in the container 42 is fed to the bioparticle-capturing module 10. The DNaseI decomposes DNAs fixing the above-described antibodies to the above-described substrate, and the target cells to which the above-described antibodies bind are released from the above-described substrate. Then, the target cells released from the substrate flow out through the outlet 13 and are recovered into the reservoir 20.

To perform the above-described recovery, for example, the pump P1 is driven with the valves V0.3, V1.4, and V2 open. During the driving, the other valves may be closed, or the other pumps need also not be driven.

The above-described driving causes the DNaseI-containing buffer to flow from the inlet 11 of the bioparticle-capturing module 10 into the container 12 through the channels 52 and 60. The DNaseI decomposes DNAs fixing the above-described antibodies to the above-described substrate, and the target cells are released from the above-described substrate. The released cells flow out through the outlet 13 and are recovered into the reservoir 20 through the channels 61, 62, and 63. Similarly to the above-described released cells, the DNaseI-containing buffer is also recovered into the reservoir 20. Thus, also in the hollow fiber membrane treatment step described below, the DNaseI exerts an effect.

### (3-4) Hollow Fiber Membrane Treatment Step

### (3-4-1) Concentration Adjustment Treatment

In the hollow fiber membrane treatment step S104, the target cells in the reservoir 20 are flowed through the hollow fiber membrane module 30. This may adjust the concentration of the target cells, for example, allowing the concentration of the target cells to be increased.

For the above-described concentration adjustment, for example, with the valves V4 and V5 open, the pumps P2 and P3 are driven. During the driving, the other valves may be closed, or the other pumps need also not be driven.

The above-described driving causes the target cell-containing liquid in the reservoir 20 to flow from the inlet 31 of the hollow fiber membrane module 30 into the container 32 through the channel 64. Then, the target cell-containing liquid having passed through the container 32 flows out through the outlet 33 and returns to the reservoir 20 through the channel 65. This causes the hollow fiber membrane in the container 32 to concentrate the target cells.

The waste resulting from the above-described concentration (permeate obtained by the hollow fiber membrane treatment) flows out through the outlet 34 for waste, and is recovered into the waste container 45 through the channels 68 and 55.

### (3-4-2) Solvent Replacement Treatment

In the hollow fiber membrane treatment step S104, a binding treatment in which the antibodies (for example, fluorescently labelled antibodies) are bound to the target cells may be executed. To execute the binding treatment, a solvent holding the target cells is preferably replaced with a solvent suitable for the binding treatment. Thus, for the binding, a solvent of the target cell-containing liquid is replaced with a staining buffer corresponding to a binding auxiliary liquid. For the solvent replacement, for example, diafiltration using the hollow fiber membrane module can be performed.

For the above-described solvent replacement, for example, the pump P1 is driven with the valves V1.1 and V2 open. Thus, a staining buffer is fed into the reservoir 20, increasing the rate of the staining buffer in the target cell-containing liquid.

Concurrently with the driving of the pump P1 with the above-described valves 1.1 and V2 open, the pumps P2 and P3 are driven with the valves V4 and V5 open. The driving of the above-described pumps P2 and P3 causes the target cell-containing liquid in the reservoir 20 to flow from the inlet 31 of the hollow fiber membrane module 30 into the container 32 through the channel 64. Then, the target cell-containing liquid having passed through the container 32 flows out through the outlet 33 and returns to the reservoir 20 through the channel 65. This causes the hollow fiber membrane in the container 32 to concentrate the target cells. The waste resulting from the above-described concentration flows out through the outlet 34 for waste, and is recovered into the waste container 55 through the channels 68 and 55.

By the driving of the pump P1 with the above-described valves V1.1 and V2 open and the driving of the pumps P2 and P3 with the valves V4 and V5 open, the solvent of the target cell-containing liquid is replaced with the staining buffer.

Note that, in the above description, the driving of the pump P1 with the above-described valves V1.1 and V2 open and the driving of the pumps P2 and P3 with the above-described valves V4 and V5 open are concurrent but that the two driving operations may be sequential and may further sequentially be repeated.

### (3-4-3) Dead Volume Recovery Treatment

The hollow fiber membrane treatment has what is generally called a dead volume problem as described below. That is, in the hollow fiber membrane treatment, treatment, for example, concentration or diafiltration, is executed with circulation of a liquid that contains hollow fiber membrane columns. Thus, when the treatment is complete, the sample remains in the channels other than the recovery container and in the hollow fiber membrane module. The amount of the remaining sample is a dead volume. For example, also in the sample preparation system depicted in FIG. 3, when the solvent replacement treatment described above in (3-4-2) is complete, the cell-containing liquid remains in the channel 64, the hollow fiber membrane module 30, and the channel 65. The amount of the remaining cell-containing liquid is a dead volume.

The dead volume problem is not manifested in a case where the hollow fiber membrane treatment causes a large amount of the target substances to be recovered into the recovery container. However, in bioparticle analysis, for example, flow cytometry, a relatively small amount of sample is used in many cases, for example, and the amount is approximately 5 mL. The amount is smaller than the amount of recovery assumed in general hollow fiber membrane treatment, and is, for example, smaller than the amount of recovery by an order of magnitude. Thus, in bioparticle analysis, the dead volume in the hollow fiber membrane treatment is desirably as small as possible.

In addition, in bioparticle analysis, rare cells, for example, certain particular immune cells, are to be analyzed. Thus, also to obtain an appropriate number of cells, the dead volume in the hollow fiber membrane treatment is desirably as small as possible.

The inventors have found that the dead volume problem can be solved by the branching channel 66 branching off from the circulating channel through which the bioparticles circulate between the reservoir 20 and the hollow fiber membrane module 30, the branching channel 66 leading to the reservoir 20.

For example, the operation below allows the dead volume resulting from the solvent replacement treatment in (3-4-2) described above to be recovered into the reservoir 20.

First, the pump P1 is driven with the valves V1.1, V3, and V4 open. During the driving, the other valves may be closed, or the other pumps need also not be driven. Thus, the target cell-containing liquid is recovered into the reservoir 20, the target cell-containing liquid being present from a connection portion between the branching channel 66 and the channel 65 (connection portion between the valves V4 and V5) to the terminal of the channel 64 in the reservoir 20.

Then, with the pump P1 open and with the valves V1.1, V3, and V5 open, the pump P2 is driven in the opposite direction (that is, in such a manner that the liquid flows through the channel 65, the hollow fiber membrane module 30, and the channel 64 in this order). Thus, of the channel 65, the target cell-containing liquid that is present from the connection portion between the branching channel 66 and the channel 65 (connection portion between the valves V4 and V5) to the outlet 33 of the hollow fiber membrane module 30, the target cell-containing liquid that is present in the hollow fiber membrane module 30, and the target cell-containing liquid that is present in the channel 64 (that is, the target cell-containing liquid in the channel from the inlet 31 of the hollow fiber membrane module 30 into the reservoir 20) are recovered into the reservoir 20,.

In driving of the above-described pump P2 in the opposite direction, preferably, the pump P3 can be driven in the opposite direction (that is, in such a manner that the liquid flows from the channel 68 into the container 32 of the hollow fiber membrane module 30). The driving is performed to urge detachment of cells from the hollow fiber membrane. The driving may be performed, for example, at low speed. In addition, the driving may be performed in a short time (for example, 0.1 to 5 seconds, particularly, 0.5 to 3 seconds). For example, the shorttime driving can be performed once or more, for example, approximately once to 10 times, particularly, approximately twice to five times. Driving the pump P3 in such a manner makes the cells more likely to be detached from the hollow fiber membrane, enabling an increase in the number of cells recovered into the reservoir 20.

### (3-4-4) Binding Treatment for Binding Antibodies to Cells

As described above, in the hollow fiber membrane treatment step S104, binding treatment in which the antibodies are bound to the cells may be executed. For the binding treatment, the antibody-containing liquid in the container 44 is introduced into the reservoir 20. Then, incubation is performed to bind the antibodies to the cells. Incubation conditions (for example, time and temperature) may be selected as appropriate by the person skilled in the art.

To introduce the above-described antibody-containing liquid into the reservoir 20, for example, the pump P1 is driven with the valves V1.2 and V2 open. During the driving, the other valves may be closed, or the other pumps need also not be driven. The driving causes the antibody-containing solution in the container 44 to be fed into the reservoir 20 through the channels 54 and 63. Then, the above-described incubation is performed. Note that, for the above-described incubation, for example, the temperature of the reservoir 20 may be maintained at approximately 4°C.

### (3-4-5) Cell Concentration Treatment

After the cell concentration treatment described above in (3-4-4), surplus antibodies are preferably removed. The removal may be performed, for example, by treatment similar to the solvent replacement treatment described above in (3-4-2), and the antibody removal treatment can be executed efficiently by increasing the cell concentration in the reservoir, that is, concentrating the cells, before the treatment. Thus, concentration treatment is executed as described above in (3-4-1).

For the concentration treatment, for example, the pumps P2 and P3 are driven with the valves V4 and V5 open. During the driving, the other valves may be closed, or the other pumps need also not be driven.

The above-described driving causes antibody-labelled target cell-containing liquid in the reservoir 20 to flow from the inlet 31 of the hollow fiber membrane module 30 into the container 32 through the channel 64, and the target cell-containing liquid having passed through the container 32 flows out through the outlet 33 and returns to the reservoir 20 through the channel 65. Thus, the hollow fiber membrane in the container 32 concentrates the antibody-labelled target cells. For example, a sample of approximately 5 ml is used for the bioparticle analysis such as flow cytometry, and thus, in a case where the sample prepared by the sample preparation system according to the present technology is used for such bioparticle analysis as described above, the sum of the amount of liquid in the reservoir resulting from the concentration treatment and the amount of the dead volume remaining in the channels can be adjusted to such an amount as described above.

### (3-4-6) Surplus Antibody Removal Treatment

As described above in (3-4-5), surplus antibodies are preferably removed after the cell labelling treatment. Thus, solvent replacement treatment using a staining buffer is executed on the target cell-containing liquid resulting from the concentration treatment described above in (3-4-5). The solvent replacement treatment may be executed, for example, as described above in (3-4-2).

Specifically, as described above in (3-4-2), the driving of the pump P1 with the valves V1.1 and V2 open and the driving of the pumps P2 and P3 with the valves V4 and V5 open can be sequentially or concurrently performed.

By the driving, the target cell-containing liquid is subjected to diafiltration using the staining buffer, removing surplus antibodies from the target cell-containing liquid.

### (3-4-7) Concentration Adjustment Treatment

After the antibody removal in (3-4-6) described above, the target cell concentration of the target cell-containing liquid may be adjusted. The adjustment of the concentration may be, for example, dilution treatment for reducing the concentration or may be concentration treatment for increasing the concentration. The target cell concentration can be selected as appropriate depending on bioparticle analysis to which the sample prepared is subjected.

For example, to execute dilution treatment, the pump P1 may be driven with the valves V1.1 and V2 open. Thus, the staining buffer is fed into the reservoir 20 through the channels 53 and 63, allowing the target cell concentration to be reduced.

Note that, in the dilution treatment, the valves V4 and V5 may be opened and the pump P2 may be driven. Thus, the cells in the target cell-containing liquid can be distributed uniformly. In addition, the driving allows pores in the hollow fibers to be prevented from being clogged with cells.

In addition, to execute concentration treatment, the pumps P2 and P3 are driven with the valves V4 and V5 open, as described above in (3-4-5). During the driving, the other valves may be closed, or the other pumps need also not be driven.

In the adjustment of the concentration, the concentration may be measured with use of the analysis apparatus 90 that analyzes the liquid in the reservoir 20. The analysis apparatus 90 may be an analysis apparatus that performs, for example, turbidity measurement or fluorescence measurement, and the turbidity or fluorescence measured can determine the concentration of the target cells. Depending on the measured concentration, start, continuance, or end of the concentration adjustment treatment (dilution treatment or concentration treatment) can be determined.

### (3-4-8) Dead Volume Recovery Treatment

After the concentration adjustment treatment described above in (3-4-7), the dead volume recovery treatment can be executed again. This allows recovery of the dead volume remaining in the channels and the hollow fiber membrane module after the concentration adjustment treatment. The dead volume treatment may be executed as described above in (3-4-3).

The treatment as described above prepares a target cell-containing liquid sample. The sample prepared may be recovered into a container outside the reservoir 20, for example, via the channel 67, and the sample can be used for the subsequent bioparticle analysis treatment with use of the container. Alternatively, the reservoir 20 itself may be used as a container allowing the sample to be used for the subsequent bioparticle analysis treatment.

### (3-5) Modification

In the treatment described above, first, bioparticle-capturing module treatment is executed, and then hollow fiber membrane module treatment is executed.

In the present technology, for example, a hollow fiber membrane treatment step (hereinafter also referred to as a "first hollow fiber membrane treatment step") may first be executed, and the bioparticle capturing step may then be executed. Further, after these steps, a hollow fiber membrane treatment step (hereinafter also referred to as a "second hollow fiber membrane treatment step") may be executed. These steps may be executed by the sample preparation system 1 described above with reference to FIGS. 1 and 3, but may be executed by, for example, a sample preparation system 3 depicted in FIG. 13. With reference to FIG. 13, the sample preparation system 3 will be described, and then an example of sample preparation treatment by the sample preparation system 3 will be described with reference to the flow diagram in FIG. 14.

The sample preparation system 3 depicted in FIG. 13 has a configuration corresponding to the sample preparation system 1 subjected to the following changes.

In the sample preparation system 1, the container 40 for feeding the cell-containing liquid is connected to the channel 60 via the channel 50. However, in the sample preparation system 3, the container 40 is connected to the channel 63 via the channel 56. In addition, a valve V6 is provided on the channel 56. Opening or closing the valve V6 enables or disables feeding, to the reservoir 20, of the cell-containing liquid (Sample) contained in the container 40.

In the sample preparation system 1, the channel 60 is connected to the container 40. However, in the sample preparation system 3, the channel 60 is not connected to the container 40 but to the channel 65, and particularly, to a portion of the channel 65 between the valves V4 and V5. In addition, a valve V8 is connected to a portion of the channel 60 between a point where the channel 60 is connected to the channel 65 and a point where the channel 60 is connected to the channel 51.

In the sample preparation system 1, the container 43 contains the staining buffer, whereas, in the sample preparation system 3, the container 43 contains the staining buffer that contains DNaseI.

In addition to the container 40 connected to the channel 63 as described above, a container 46 containing a staining buffer that contains no DNaseI is connected to the channel 63 via the channel 57. A valve V7 is provided on the channel 57. Opening or closing the valve V7 enables or disables feeding, to the reservoir 20, of the above-described no-DNaseI-containing staining buffer in the container 40.

FIG. 14 depicts an example of a flowchart of the sample preparation treatment executed by the sample preparation system 3. The treatment may be executed, for example, as follows.

### (3-5-1) Priming Step

In a priming step S201, as described above in (3-1), the channel through which the cell-containing liquid passes is filled with, for example, the wash buffer or the staining buffer. In the priming step S201, the bioparticle-capturing module 10, the reservoir 20, and the hollow fiber membrane module 30 can also be filled with the wash buffer or the staining buffer. The control of the valves and the pumps in the priming step may be performed, for example, as described above in (3-1).

### (3-5-2) Feeding Step

In a feeding step S202, the reservoir 20 is fed with the cell-containing liquid in the container 40 and the DNaseI-containing staining buffer in the container 43. For the feeding described above, for example, the pump P1 is first driven with the valves V6 and V2 open, and the pump P1 is then driven with the valves V1.1 and V2 open. The former driving causes the cell-containing liquid to be introduced into the reservoir 20, and then the latter driving causes the above-described staining buffer to be fed to the reservoir 20.

### (3-5-3) First Hollow Fiber Membrane Treatment Step

In a first hollow fiber membrane treatment step S203, the hollow fiber membrane module 30 can be used to adjust the concentration of the cell-containing liquid in the reservoir 20. Then, after the adjustment, the cells in the reservoir 20 are labelled with the antibodies in the container 44. To perform the labeling, the hollow fiber membrane treatment step S203 may include, for example, "(3-4-1) Concentration Adjustment Treatment," "(3-4-2) Solvent Replacement Treatment," "(3-4-3) Dead Volume Recovery Treatment," "(3-4-4) Binding Treatment for Binding Cells to Antibodies," and "(3-4-5) Cell Concentration Treatment," described above in (3-4). These treatment operations may be as described above in (3-4), and the description of the treatment operations applies to the present modification.

After the above-described cell concentration treatment, the surplus antibody removal treatment in the reservoir 20 may be executed. The surplus antibody removal treatment may be executed by replacing the solvent in the reservoir 20. Specifically, driving of the pump P1 with the valves V7 and V2 open and driving of the pumps P2 and P3 with the valves V4 and V5 open may be concurrently or sequentially performed. Thus, the liquid in the reservoir 20 is replaced with the no-DNaseI-containing staining buffer in the container 46. That is, the liquid that contains cells contains no DNaseI.

### (3-5-4) Capturing Step

### (3-5-4-1) Capturing Treatment

In a capturing step S204, the cell-containing liquid in the reservoir 20 is fed to the bioparticle-capturing module 10. As described above in (3-2-1), the container 12 of the bioparticle-capturing module 10 contains antibodies that bind specifically to the target cells and that are in the solid phase, as described above. Thus, the target cells bind to the antibodies and are thus captured into the container 12.

For feeding of the above-described cell-containing liquid to the bioparticle-capturing module 10, for example, the pump P3 is driven with the valves V4, V8, and V0.4 open. During the driving, the other valves may be closed, or the other pumps need also not be driven.

The above-described driving causes the cell-containing liquid in the reservoir 20 to flow from the inlet 11 of the bioparticle-capturing module 10 into the container 12 through the channel 60 (more specifically, flow from the reservoir 20 though a portion of the channel 60 in which the valve V4 is disposed and through a portion of the channel 60 in which the valve V8 is disposed, in this order). Thus, the target cells are captured by the antibodies in the container 12, while the nontarget cells pass through the container 12, flow out through the outlet 13, and are then recovered into the waste container 45 through the channels 61, 69, and 55, as described above.

### (3-5-4-2) Surplus Cell Removal Treatment

In the operation described above in (3-5-4-1), the surplus cell removal treatment described above in (3-2-2) may be executed. The operation of the valves and the pumps for the removal treatment may be performed as described above in (3-2-2).

### (3-5-5) Recovery Step

In a recovery step S205, the target cells in the container 12 of the bioparticle-capturing module 10 are released from the substance that captures the bioparticles and recovered into the reservoir 20 as described above in (3-3). To perform the above-described release, the DNaseI-containing buffer in the container 42 is fed to the bioparticle-capturing module 10. The DNaseI decomposes DNAs fixing the above-described antibodies to the above-described substrate, releasing, from the above-described substrate, the target cells to which the above-described antibodies are bound. Then, the target cells released from the substrate flow out through the outlet 13 and are recovered into the reservoir 20.

To perform the above-described recovery, for example, the pump P1 is driven with the valves V0.3, V1.4, and V2 open. During the driving, the other valves may be closed, or the other pumps need also not be driven.

The above-described driving causes the DNaseI-containing buffer to flow from the inlet 11 of the bioparticle-capturing module 10 into the container 12 through the channels 52 and 60. The DNaseI decomposes DNAs fixing the above-described antibodies to the above-described substrate, releasing the target cells from the above-described substrate. The released cells flow out through the outlet 13 and are recovered into the reservoir 20 through the channels 61, 62, and 63. Similarly to the above-described released cells, the DNaseI-containing buffer is also recovered into the reservoir 20. Thus, the DNaseI exerts an effect also in the next treatment.

### (3-5-6) Second Hollow Fiber Membrane Treatment Step

A second hollow fiber membrane treatment step S206 can include wash treatment for the cells in the cell-containing liquid that contains the target cells recovered into the reservoir and cell concentration treatment for concentrating the target cells. The cell-containing liquid obtained by the above-described second hollow fiber membrane treatment step may be treated as a sample prepared by the system of the present technology. The wash treatment may be executed by, for example, performing the same operations as those of the solvent replacement treatment described above in (3-4-2). The cell concentration treatment may be executed, for example, as described above in (3-4-5).

Note that the target cell-containing liquid recovered into the reservoir 20 in (3-5-5) described above may be treated as a sample prepared by the system of the present technology, without execution of the second hollow fiber membrane treatment step.

### (4) Example of Treatment Using Bioparticle-Capturing Module

The bioparticle-capturing module includes a substrate to which the substance that captures the bioparticles is fixed, as described above. The substance that captures the bioparticles captures the target bioparticles. The nontarget cells can be disposed of, for example, by washing, without being captured. Thus, the target bioparticles can be selectively fed to the reservoir. A specific example of the operation of the bioparticle-capturing module will be described below.

### (4-1) Configuration Example and Operation Example of Bioparticle-Capturing Module

With reference to FIG. 4, the capturing and releasing of the target cells by the bioparticle-capturing module will be described. As depicted in FIG. 4A, the bioparticle-capturing module includes a substrate S to which the substance that captures the bioparticles is fixed. The substrate S may be a fibrous substrate as depicted in FIG. 4A or may not be fibrous. For example, the substrate S may be a porous substrate or a substrate shaped like beads.

The substance that captures the bioparticles is fixed to the substrate S. Preferably, the substance that captures the bioparticles is fixed to the substrate via nucleic acids. Thus, the target cells can be released by the above-described nucleolytic substance.

The substrate S may be packed into the bioparticle-capturing module with a gap of from 100 to 1000 pm, particularly, from 200 to 800 µm or from 300 to 700 pm, and more particularly, approximately 500 pm. The size of the gap may be set to allow the bioparticles (particularly, the cells) to easily pass through.

The substance that captures the bioparticles is preferably a substance that binds to the target bioparticles, and is particularly an antibody, as described above. The type of the antibody may be selected depending on the target bioparticles, particularly, antigens on the surfaces of the target bioparticles. Thus, in the preferred embodiment of the present technology, the antibodies may be fixed to the substrate via nucleic acids.

As depicted in FIG. 4B, the bioparticle-containing liquid is fed to the bioparticle-capturing module. The bioparticle-containing liquid contains target bioparticles Pt and nontarget bioparticles Pn.

As depicted in FIG. 4C, when the bioparticle-containing liquid is caused to pass through the bioparticle-capturing module, the target particles Pt are captured by the substance that captures the bioparticles, the substance being fixed to the substrate S. On the other hand, the nontarget particles Pn are not captured by the substance that captures the bioparticles and pass through the bioparticle-capturing module. Note that, as depicted in FIG. 4C, not all the target particles may pass through the bioparticle-capturing module, with some of the target particles remaining in the bioparticle-capturing module.

To bind the bioparticles to the substance that captures the bioparticles, the bioparticles need to come into contact with the substrate S. The frequency of the contact may be controlled, for example, by flow velocity. In addition, after the bioparticle-containing liquid is fed into the bioparticle-capturing module, the bioparticle-containing liquid may be maintained in the module for a predetermined time as necessary, that is, incubation may be performed for the above-described binding. The incubation time may be selected as appropriate and can be, for example, from 10 minutes to overnight, from 10 minutes to 12 hours, from 10 minutes to five hours, from 10 minutes to three hours, from 20 minutes to two hours, or 30 to 60 minutes.

As depicted in FIG. 4D, the target particles Pt are released from the substrate S. For the release, the nucleolytic substance may be used as described above. The nucleolytic substance releases, from the substrate, the substance that captures the bioparticles and that has captured the bioparticles, and the substance that captures the bioparticles is recovered into the reservoir.

### (4-2) Test Example (Treatment of Blood with Use of Substrate to Which Substance That Captures Bioparticles Is Fixed)

In a 0.1-M MES (pH 6.0) and 0.5-M NaCl solution, an antibody-fixing solution that contained 62.5 µg/mL anti-porcine CD3 antibodies (Mouse Anti-Porcine CD3ε, clone PPT3, Southern Biotechnology Associates, Inc.), 7.5 µg/mL N-Hydroxysuccinimide (NHS) (NACALAI TESQUE, INC.), and 5.0 µg/mL 1-Ethyl-3-(3-dimethylaminopropyl)-carbodiimide HCl (EDC) (NACALAI TESQUE, INC.) was prepared. The antibody-fixing solution was left untouched for 15 minutes. After the antibody-fixing solution was left untouched as described above, 5xPBS(-) having a fourfold larger amount than the antibody-fixing solution was added to the antibody-fixing solution, and a collagen sponge (Collagen Sponge MIGHTY, sterilized, Φ5 × 3 mm, KOKEN CO., LTD.) was further added to the antibody-fixing solution. One hour later, the collagen sponge was taken out and moved to D-PBS(-). The above-described steps caused the anti-porcine CD3 antibodies to be fixed to the collagen sponge. Three collagen sponges subjected to the fixation treatment were packed in a 1-mL syringe. FIG. 5A depicts a photograph of the packed syringe. A culture medium was added to the syringe, and the inside of the syringe was washed.

After the washing, as depicted in FIG. 5B, 1 mL of whole porcine blood was added to the syringe. Then, as depicted in FIG. 5C, it was visually confirmed that the red blood cells naturally passed through the collagen sponges and dropped down. Note that a portion of the whole blood remained in part of the syringe but that a plunger was pressed to allow the remaining whole blood to pass through the collagen sponges. The above-described results confirm that the red blood cells pass through without being captured in the collagen sponges.

Subsequently, hydraulic pressure was applied to the syringe to recover fractions captured in the collagen sponges. The fractions recovered were analyzed by flow cytometry. In addition, the above-described whole blood was also analyzed by flow cytometry. FIG. 5D indicates analysis results for the above-described whole blood, and FIG. 5E indicates analysis results for the above-described fractions. As indicated in FIG. 5D, for the whole blood, substantially no cells were confirmed in a white blood cell gate. On the other hand, for the fractions, cells were confirmed in 40% of the detection targets in the white blood cell gate. The above-described results confirm that the collagen sponges to which the antibodies were fixed allow the percentage of white blood cells to be increased.

### (4-3) Another Configuration Example and Another Operation Example of Bioparticle-Capturing Module

In the present technology, the substance that captures the bioparticles may be a substance that captures the bioparticles via a different substance, and may be a substance that captures the bioparticles, for example, via a bioparticle-binding substance. With reference to FIG. 6, description will be given of a configuration example and an operation example of a bioparticle-capturing module including a substrate to which the substance that captures the bioparticles via the different substance is fixed.

As depicted in FIG. 6A, the bioparticle-capturing module includes the substrate S to which the substance that captures the bioparticles is fixed. The substrate S may be a fibrous substrate as depicted in FIG. 6A or may not be fibrous. For example, the substrate S may be a porous substrate or may be a substrate shaped like beads.

The substance that captures the bioparticles is fixed to the substrate S. Preferably, the substance that captures the bioparticles is fixed to the substrate via nucleic acids. Thus, the target cells can be released by the above-described nucleolytic substance.

The substrate S may be packed into the bioparticle-capturing module with a gap of from 100 to 1000 pm, particularly, from 200 to 800 µm or from 300 to 700 pm, and more particularly, approximately 500 pm. The size of the gap may be set to allow the bioparticles (particularly, the cells) to easily pass through.

The substance that captures the bioparticles via the different substance may be a substance that binds to a bioparticle-binding substance as described above, for example, an antibody-binding protein. The antibody-binding protein may be, for example, any one or two or more of a protein A, a protein G, a protein L, and a protein A/G. As described above, in the preferred embodiment of the present technology, the antibody-binding protein may be fixed to the substrate via nucleic acids.

As depicted in FIG. 6B, the antibody-containing liquid is fed to the bioparticle-capturing module. As the antibody-containing liquid passes through the module, antibodies AB corresponding to the bioparticle-binding substance bind to the antibody-binding protein. Thus, as depicted in FIG. 6C, the antibodies AB are fixed to the substrate S. The antibodies AB may bind specifically to the target particles.

As depicted in FIG. 6D, the bioparticle-containing liquid is fed to the bioparticle-capturing module. The bioparticle-containing liquid contains the target bioparticles Pt and the nontarget bioparticles Pn.

As depicted in FIG. 6E, when the bioparticle-containing liquid is caused to pass through the bioparticle-capturing module, the target particles Pt are captured by the antibodies AB fixed to the substrate S. On the other hand, the nontarget particles Pn are not captured by the antibodies AB and pass through the bioparticle-capturing module. Note that, as depicted in FIG. 6E, not all the nontarget particles can pass through the bioparticle-capturing module and some of the nontarget particles may remain in the bioparticle-capturing module. Note that, to discharge more nontarget particles out from the module, for example, a wash step for causing the wash buffer to flow through the module may be executed.

After FIG. 6E, treatment in which the antibodies (particularly, fluorescently labelled antibodies) are bound to antigens on the surfaces of the target bioparticles Pt may be executed. For the treatment, the antibody-containing liquid is flowed through the module. The treatment may be executed, for example, antibody staining treatment. As described above, in the present technology, the antibody staining treatment may be executed in the bioparticle-capturing module.

After the treatment described With reference to FIG. 6E or 6F, the target particles Pt are released from the substrate S. For the release, the nucleolytic substance may be used as described above. The nucleolytic substance releases the antibody-binding protein from the substrate. With the release, the antibodies AB bound to the antibody-binding protein and the target bioparticles Pt bound to the antibodies AB are released from the substrate and recovered into the reservoir.

### (5) Evaluation of Effects of Nucleolytic Substance on Treatment by Hollow Fiber Membrane Module

In the sample preparation method described above in (3), in the recovery step S103, the DNaseI corresponding to the nucleolytic substance is used to release the target cells from the substrate. The nucleolytic substance is recovered into the reservoir 20 together with the target cells. Then, in the hollow fiber membrane treatment step S104, the nucleolytic substance flows through the hollow fiber membrane module 30 together with the target cells. In the present technology, the nucleolytic substance flows through the hollow fiber membrane module 30 as described above, to allow clogging in the hollow fiber membrane module 30 to be prevented.

To confirm a clogging prevention effect in the hollow fiber membrane treatment step using the nucleolytic substance, an apparatus 2 that had a configuration depicted in FIG. 7 was prepared. The apparatus includes the reservoir 20 and the hollow fiber membrane module 30 to allow the cell-containing liquid in the reservoir 20 to be concentrated. The container 43 containing the staining buffer is connected to the reservoir via the channel. The reservoir 20 and the hollow fiber membrane module 30 are as described above in (3) .

The cell-containing liquid containing the DNaseI was poured into the reservoir 20 of the apparatus 2, and then, the hollow fiber membrane module 30 was used to execute a set of treatment operations similar to the cell concentration treatment described above in (3-4-1), the solvent replacement treatment described above in (3-4-2), the dead volume recovery treatment described above in (3-4-3), and the binding treatment for binding the antibodies to the cells as described above in (3-4-4). Note that, in the binding treatment, the antibodies were manually and directly added into the reservoir 20 instead of being introduced through the channel connected to the reservoir 20. More specifically, the operation was performed as follows.

First, the components (the channels, the reservoir, and the hollow fiber membrane module) in contact with the liquids in the apparatus 2 were primed. Then, 12 mL of suspension with 1 × 10⁸ mononuclear cells was added into the reservoir 20. The hollow fiber membrane module 30 of the apparatus 2 was used to double the concentration of the cells in the suspension, and the solvent replacement was performed to replace the concentrated suspension with a DNase buffer having a fourfold larger amount than the suspension. After the solvent replacement, the DNase was added into the reservoir and circulated for approximately 10 minutes. After the solvent replacement, the dead volume recovery treatment was executed. The amount of recovery resulting from the recovery treatment was 20 mL. The binding treatment (4°C, 30 to 60 minutes) in which three types of cell staining antibodies were added into the reservoir 20 to bind the antibodies to the cells was executed. The suspension resulting from the binding treatment was concentrated by a factor of approximately four, and the solvent replacement treatment was executed with use of a staining buffer having a fivefold larger amount than the suspension. Then, the dead volume recovery treatment was executed to recover the cells. The amount of recovery resulting from the recovery treatment was 5.5 mL. Further, a small amount (approximately 2 mL) of staining buffer was circulated, and the cells were similarly recovered.

In addition, a similar set of treatment operations was performed except that the cell-containing liquid contained no DNaseI. Specifically, the treatment was executed as follows.

First, the components (the channels, the reservoir, and the hollow fiber membrane module) in contact with the liquids in the apparatus 2 were primed. Then, 12mL of suspension with 1 × 10⁸ mononuclear cells was added into the reservoir 20. The hollow fiber membrane module 30 of the apparatus 2 was used to double the concentration of the cells in the suspension, and the solvent replacement was performed to replace the concentrated suspension with a staining buffer having a fourfold larger amount than the suspension. After the solvent replacement, the dead volume recovery treatment was executed. The amount of recovery resulting from the recovery treatment was 20 mL. The binding treatment (4°C, 30 to 60 minutes) in which three types of cell staining antibodies were added into the reservoir 20 to bind the antibodies to the cells was executed. The suspension resulting from the binding treatment was concentrated by a factor of approximately four, and the solvent replacement treatment was executed with use of a staining buffer having a fivefold larger amount than the suspension. Then, the dead volume recovery treatment was executed to recover the cells. The amount of recovery resulting from the recovery treatment was 5.5 mL. Further, a small amount (approximately 2 mL) of staining buffer was circulated, and the cells were similarly recovered.

In the case of the use of the cell-containing liquid containing the DNaseI, the execution of the above-described set of treatment operations did not cause clogging in the hollow fiber membrane module 30 and in the circulating channel connecting the hollow fiber membrane module 30 and the reservoir 20 to each other.

On the other hand, in the case of the use of the cell-containing liquid containing no DNaseI, a cellular aggregate was confirmed in the reservoir 20 after the solvent replacement treatment as depicted in FIG. 8. In addition, clogging occurred in the hollow fiber membrane module 30.

The above-described results indicate that the use of the nucleolytic substance, for example, the DNaseI, enables cell aggregation to be prevented, allowing prevention of possible clogging in the hollow fiber membrane module. Thus, the present technology preferably uses the nucleolytic substance. For example, the sample preparation system of the present technology may be configured to allow the nucleolytic substance to reach the reservoir or the hollow fiber membrane module through the bioparticle-capturing module.

### (6) Evaluation of Antibody Staining Using Hollow Fiber Membrane Module

In the sample preparation method described above in (3), the binding treatment for binding the antibodies to the cells is executed in the hollow fiber membrane treatment step S104. The binding treatment is also referred to as antibody staining treatment. Evaluation was conducted as follows to check whether the cells were stained with the antibodies by the antibody staining treatment using the hollow fiber membrane module.

First, the apparatus described above in (5) with reference to FIG. 7 was prepared. The apparatus was used to execute, on PBMCs (peripheral blood mononuclear cells), the solvent replacement treatment described above in (3-4-2) and the binding treatment for binding the antibodies to the cell as described above in (3-4-4). In addition, the antibody staining treatment was executed on the above-described PBMCs with use of the same antibodies. The materials and specific operational procedure used in these treatment operations are as described below. Note that FIG. 12 also depicts a schematic diagram of the operational procedure.

### (6-1) Experimental Materials

Hollow fiber membrane module: C02-E65U-07-N (Repligen Corporation, non-sterilized, pore size: 0.65 um, membrane material: mPES)
Pressure sensor that measures the pressure of the hollow fiber membrane module: ACPM-799-01N (Repligen Corporation, made of polysulfone)
Tubes constituting the channels: Pharmapure #14
Frozen PBMCs: five vials each containing 1 × 10⁸ cells
Antibodies: PE labelled anti CD3 antibodies, FITC labelled anti CD4 antibodies, and VioBlue labelled anti CD8 antibodies

### (6-2) Preparation of PBMCs and Manual Staining

Step 1-1. In a water bath at 37°C, the frozen PBMCs in each vial were heated for approximately two minutes.

Step 1-2. When only a small amount of ice remained in each vial, 5 mL of TM buffer containing 10% human albumin was added into each vial and pipetted, and the contents of each vial were transferred to a 50-mL tube.

Step 1-3. 1 mL of the above-described TM buffer was poured in an empty vial and pipetted, and the resultant TM buffer was poured into each vial in 2.

Step 1-4. The above-described TM buffer was added in such a manner that the total amount in the 50-mL tube was 13 mL, and a 40-pm cell strainer was applied to the TM buffer.

Step 1-5. 1 mL of the permeate passed through the cell strainer was recovered, and more than 100 µL of the 1-mL permeate was stained with PI. Then, the numbers of white blood cells and cells were measured with use of a cell analyzer (SP-6800, Sony Corporation) and a multichannel automatic blood cell counter (pocH-100I, Sysmex Corporation). The number of white blood cells was 93 × 10²/µL, and the number of cells was 9 × 10⁶.

Step 1-6. Of the permeate passed through the cell strainer, the remaining 12 mL was subjected to treatment using the above-described apparatus including the above-described hollow fiber membrane module. The treatment will be described below in <Staining Treatment Using Hollow Fiber Membrane Module>.

Step 1-7. Of the 1-mL permeate of Step 1-5. described above, the remaining 200 g was centrifuged for 10 minutes at the room temperature.

Step 1-8. After the centrifuging in Step 1-7. described above, supernatant was removed with pellets remaining, and tapping was performed.

Step 1-9. 200 uL of wash buffer (WB) containing 200 U/mL of DNaseI was added to the above-described pellets, and the pellets were then mixed with the wash buffer by inverting the vial.

Step 1-10. Into the sample resulting from Step 1-9. described above, a staining buffer was added in such a manner that the total amount was 1.4 mL (44 × 10² white blood cells/pL, 6 × 10⁶ cells) .

Step 1-11. Four 100-µL samples were taken from the 1.4-mL sample prepared in Step 1-10. described above. Of the four 100-µL samples, two samples were stained with use of three antibodies (PE labelled anti CD3 antibodies, FITC labelled anti CD4 antibodies, and VioBlue labelled anti CD8 antibodies), and the samples were immediately manually washed. Subsequently, the samples were stained with PI (Propidium Iodide), and then analyzed with use of the above-described cell analyzer.

Step 1-12. Of the four 100-µL samples prepared in Step 1-11. described above, the remaining two samples were also stained with use of the above-described antibodies for one hour at the room temperature or 4°C. After the staining, treatment using the above-described apparatus including the above-described hollow fiber membrane module was executed, and then, the samples were manually washed. Subsequently, the samples were stained with PI (Propidium Iodide), and then analyzed with use of the above-described cell analyzer.

### (6-3) Staining Treatment Using Hollow Fiber Membrane Module

Step 2-1. Of the permeate described above in 6 of (6-2) described above, a 12-mL solvent was replaced with a staining buffer by the above-described apparatus including the above-described hollow fiber membrane module. The amount of the sample resulting from the solvent replacement was 20 mL. The 40-pm cell strainer was applied to the 20-mL sample. 20 mL of the permeate passed through the cell strainer was divided into 1 mL and 19 mL. Each of the three types of the above-described antibodies was added in 237.5 µL into the 19-mL sample. The antibody addition was performed in the 50-mL tube.

Step 2-2. After the antibody addition in Step 2-1. described above, incubation was performed in a cool and dark place for one hour.

Step 2-3. The sample incubated in Step 2-2. described above was subjected to concentration treatment by the hollow fiber membrane module with use of the above-described apparatus. The concentration treatment increased the concentration of the cells in the cell suspension by a factor of 4.

Step 2-4. After Step 2-3. described above, the above-described apparatus was used to execute wash treatment for removing surplus antibodies with use of the staining buffer. The wash treatment was solvent replacement treatment using a staining buffer having a five times larger amount than the concentrated cell suspension.

Step 2-5. After Step 2-4. described above, recovery treatment was executed to recover the dead volume in the circulating channel between the reservoir 20 and the hollow fiber membrane module 30 and in the hollow fiber membrane module 30. After the recovery by the recovery treatment, the staining buffer was circulated, and the cells were similarly recovered.

Step 2-6. For the total of the sample in the reservoir obtained by the wash treatment in Step 2-4. described above and the amount of recovery obtained in the recovery treatment in Step 2-5., the number of cells were measured with use of the multichannel automatic blood cell counter (pocH-100I, Sysmex Corporation). In addition, the sample was stained with PI (Propidium Iodide), and then analyzed with use of the above-described cell analyzer.

FIG. 9 illustrates results of analyzer analysis of the cells manually stained as described above in (6-2) and results of analyzer analysis in Step 2-6. in (6-3) described above. As illustrated in FIG. 9, each type of antibodies had a higher rate of positive cells for the antibodies from among the cells stained with use of the hollow fiber membrane module than the manually treated antibodies. This indicates that the staining treatment using the hollow fiber membrane module has a higher staining efficiency than the manual operation. It was also confirmed that the staining treatment using the hollow fiber membrane module involves the concentration treatment and the wash treatment but allows for appropriate staining.

In addition, FIG. 10 illustrates, for the cells stained with use of the hollow fiber membrane module described above in (6-3), results of measurement of the number of cells by the above-described blood cell counter. As illustrated in FIG. 10, in Step 1-4., the amount of liquid was 13 mL, and the number of white blood cells was 93 × 10²/µL. Then, after the wash treatment in Step 2-4., the amount of liquid was 5.3 mL, and the number of white blood cells was 127 × 10²/µL. The recovery rate of white blood cells was 80.5%. The recovery rate is the ratio, to the number of white blood cells that have not yet been subjected to the concentration treatment and the wash treatment, of the number of white blood cells that have been subjected to these treatment operations.

In addition, by adding the amount of recovery obtained from the recovery treatment in Step 2-5. to the amount of recovery resulting from the wash treatment in Step 2-4., the recovery rate is further increased to 85.4%. This indicates that, by adding the staining buffer and performing washing, the recovery rate can further be increased.

Note that, when manual staining treatment was executed without use of the hollow fiber membrane module, the recovery rate was 75%. This indicates that the use of the hollow fiber membrane module allows acquisition of results comparable to those obtained from the manual operation.

In the treatment using the hollow fiber membrane module as described above in (6-3), the DNaseI is added to the staining buffer used in Step 2-1., as described above. In Step 2-1., no cellular aggregate was observed in the sample resulting from the use of the cell strainer and execution of the solvent replacement.

The same treatment as that using the hollow fiber membrane module described above in (6-3) was executed except that no DNaseI was added. Results of the treatment confirmed cellular aggregates in the cell strainer in Step 2-1. as depicted in photographs in A and B of FIG. 11. In addition, cellular aggregates were also confirmed in the sample resulting from the solvent replacement in Step 2-1., as depicted in the photographs in C and D of FIG. 11.

These results indicate that the nucleolytic substance enables prevention of possible cellular aggregates in the treatment using the hollow fiber membrane module, allowing, for example, clogging and the like to be suppressed. In addition, the results also indicate that suppression of possible cellular aggregates enables the recovery rate of cells to be increased.

### 2. Second Embodiment (Sample Preparation Method)

The present technology also provides a sample preparation method including a capturing step of capturing bioparticles with use of a substrate to which a substance that captures the bioparticles is fixed, a recovery step of recovering, into a reservoir, the bioparticles released from the above-described substrate, and a hollow fiber membrane treatment step of causing the bioparticles recovered into the above-described reservoir to flow through the hollow fiber membrane module. The sample preparation method may be executed, for example, with use of the sample preparation system according to the present technology described above in 1, but may also be executed with use of another system.

The capturing step, the recovery step, and the hollow fiber membrane treatment step described above are respectively as described above in (3-2), (3-3), and (3-4) in 1, and the description also applies to the present embodiment.

In addition, the sample preparation method of the present technology may further include a priming step as the one described above in (3-1) in 1.

Note that the present technology can also be configured as described below.
[1] A sample preparation system including:
   a bioparticle-capturing module including a substrate to which a substance that captures bioparticles is fixed;
   a reservoir into which the bioparticles released from the substrate are recovered; and
   a hollow fiber membrane module through which the bioparticles in the reservoir are flowed.
[2] The sample preparation system according to [1], in which
   the sample preparation system is configured to allow a nucleolytic substance to be fed to the bioparticle-capturing module.
[3] The sample preparation system according to [1] or [2], in which
   the substance that captures the bioparticles is fixed to the substrate via nucleic acids.
[4] The sample preparation system according to [2], in which
   the sample preparation system is configured to allow the nucleolytic substance to reach the reservoir or the hollow fiber membrane module through the bioparticle-capturing module.
[5] The sample preparation system according to any one of [1] to [4], in which
   the sample preparation system is configured to allow a bioparticle-containing liquid to be fed to the bioparticle-capturing module and to allow a particle-binding substance bound to the bioparticles to be fed to the reservoir.
[6] The sample preparation system according to [5], in which
   the particle-binding substance is an antibody.
[7] The sample preparation system according to [5], in which
   the bioparticles are cells.
[8] The sample preparation system according to any one of [1] to [7], including:
   a circulating channel through which the bioparticles are circulated between the reservoir and the hollow fiber membrane module.
[9] The sample preparation system according to [8], further including:
   a branching channel branching off from the circulating channel and leading to the reservoir.
[10] The sample preparation system according to any one of [1] to [9], further including:
   an analysis apparatus that analyzes contents of the reservoir.
[11] The sample preparation system according to [10], in which
   the sample preparation system controls a flowthrough operation in which the bioparticles are flowed through the hollow fiber membrane module, on the basis of an analysis result from the analysis apparatus.
[12] The sample preparation system according to any one of [1] to [11], in which
   a pump is provided on a channel leading from the bioparticle-capturing module to the reservoir.
[13] The sample preparation system according to [12], in which
   the pump includes a tube pump.
[14] The sample preparation system according to [12] or [13], in which
   the sample preparation system is configured to allow the bioparticles released from the substrate to be fed to the reservoir by driving of the pump.
[15] The sample preparation system according to any one of [12] to [14], in which
   the sample preparation system is configured to allow a particle-binding substance bound to the bioparticles to be fed to the reservoir by driving of the pump.
[16] The sample preparation system according to any one of [1] to [15], in which
   a bioparticle circulation pump is provided on a channel leading from the reservoir to the hollow fiber membrane module.
[17] The sample preparation system according to any one of [1] to [16], in which
   the hollow fiber membrane module includes a discharge channel through which a liquid separated from the bioparticles is discharged, and a discharge pump is provided on the discharge channel.
[18] The sample preparation system according to any one of [1] to [17], in which
   the sample preparation system is configured to prevent a sample containing the bioparticles from communicating fluidly with an external environment.
[19] A sample preparation method including:
   a capturing step of capturing bioparticles with use of a substrate to which a substance that captures the bioparticles is fixed;
   a recovery step of recovering, into a reservoir, the bioparticles released from the substrate; and
   a hollow fiber membrane treatment step of causing the bioparticles recovered into the reservoir to flow through a hollow fiber membrane module.

### [Reference Signs List]

1: Sample preparation system
10: Bioparticle-capturing module
20: Reservoir
30: Hollow fiber membrane module

## Claims

1. A sample preparation system comprising:
a bioparticle-capturing module including a substrate to which a substance that captures bioparticles is fixed;
a reservoir into which the bioparticles released from the substrate are recovered; and
a hollow fiber membrane module through which the bioparticles in the reservoir are flowed.

2. The sample preparation system according to claim 1, wherein
the sample preparation system is configured to allow a nucleolytic substance to be fed to the bioparticle-capturing module.

3. The sample preparation system according to claim 1, wherein
the substance that captures the bioparticles is fixed to the substrate via nucleic acids.

4. The sample preparation system according to claim 2, wherein
the sample preparation system is configured to allow the nucleolytic substance to reach the reservoir or the hollow fiber membrane module through the bioparticle-capturing module.

5. The sample preparation system according to claim 1, wherein
the sample preparation system is configured to allow a bioparticle-containing liquid to be fed to the bioparticle-capturing module and to allow a particle-binding substance bound to the bioparticles to be fed to the reservoir.

6. The sample preparation system according to claim 5, wherein
the particle-binding substance is an antibody.

7. The sample preparation system according to claim 5, wherein
the bioparticles are cells.

8. The sample preparation system according to claim 1, comprising:
a circulating channel through which the bioparticles are circulated between the reservoir and the hollow fiber membrane module.

9. The sample preparation system according to claim 8, further comprising:
a branching channel branching off from the circulating channel and leading to the reservoir.

10. The sample preparation system according to claim 1, further comprising:
an analysis apparatus that analyzes contents of the reservoir.

11. The sample preparation system according to claim 10, wherein
the sample preparation system controls a flowthrough operation in which the bioparticles are flowed through the hollow fiber membrane module, on a basis of an analysis result from the analysis apparatus.

12. The sample preparation system according to claim 1, wherein
a pump is provided on a channel leading from the bioparticle-capturing module to the reservoir.

13. The sample preparation system according to claim 12, wherein
the pump includes a tube pump.

14. The sample preparation system according to claim 12, wherein
the sample preparation system is configured to allow the bioparticles released from the substrate to be fed to the reservoir by driving of the pump.

15. The sample preparation system according to claim 12, wherein
the sample preparation system is configured to allow a particle-binding substance bound to the bioparticles to be fed to the reservoir by driving of the pump.

16. The sample preparation system according to claim 1, wherein
a bioparticle circulation pump is provided on a channel leading from the reservoir to the hollow fiber membrane module.

17. The sample preparation system according to claim 1, wherein
the hollow fiber membrane module includes a discharge channel through which a liquid separated from the bioparticles is discharged, and a discharge pump is provided on the discharge channel.

18. The sample preparation system according to claim 1, wherein
the sample preparation system is configured to prevent a sample containing the bioparticles from communicating fluidly with an external environment.

19. A sample preparation method comprising:
a capturing step of capturing bioparticles with use of a substrate to which a substance that captures the bioparticles is fixed;
a recovery step of recovering, into a reservoir, the bioparticles released from the substrate; and
a hollow fiber membrane treatment step of causing the bioparticles recovered into the reservoir to flow through a hollow fiber membrane module.
